(19) Europäisches Patentamt
European Patent Office
Office européen des brevets

(11) Veröffentlichungsnummer: **0 485 794 B1**

(12) **EUROPÄISCHE PATENTSCHRIFT**

(45) Veröffentlichungstag der Patentschrift: **09.08.95**

(51) Int. Cl.$^{6}$: **C07C 271/22**, A01N 37/44

(21) Anmeldenummer: **91118318.4**

(22) Anmeldetag: **28.10.91**

# (54) Substituierte Aminosäureamid-Derivate, deren Herstellung und Verwendung.

(30) Priorität: **10.11.90 DE 4035851**

(43) Veröffentlichungstag der Anmeldung:
**20.05.92 Patentblatt 92/21**

(45) Bekanntmachung des Hinweises auf die Patenterteilung:
**09.08.95 Patentblatt 95/32**

(84) Benannte Vertragsstaaten:
**BE CH DE ES FR GB GR IT LI NL**

(56) Entgegenhaltungen:
**EP-A- 0 236 874**
**EP-A- 0 294 667**
**EP-A- 0 374 952**

(73) Patentinhaber: **BAYER AG**
**D-51368 Leverkusen (DE)**

(72) Erfinder: **Seitz, Thomas, Dr.**
**Mozartstrasse 32**
**W-4019 Monheim (DE)**
Erfinder: **Dehne, Heinz-Wilhelm, Dr. habil.**
**Krischer Strasse 81**
**W-4019 Monheim (DE)**

**EP 0 485 794 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Aminosäureamid-Derivate und mehrere Verfahren zu deren Herstellung, sowie deren Verwendung in Schädlingsbekämpfungsmitteln.

Die erfindungsgemäßen Substanzen besitzen eine ausgezeichnete Wirkung bei der Bekämpfung von Schädlingen. Insbesondere können die erfindungsgemäßen Substanzen als Fungizide, vor allem im Pflanzenschutz, verwendet werden.

Bestimmte Aminosäureamide sind bereits bekannt, wie beispielsweise N-tert.-Butoxycarbonyl-L-leucyl-benzyl-amid (EP-A-236 874 ,EP-A-294 667).

Eine Verwendung dieser Verbindungen in Schädlingsbekämpfungsmitteln wird jedoch nicht beschrieben.

Gegenstand der vorliegenden Anmeldung sind somit neue Aminosäureamid-Derivate der allgemeinen Formel (I)

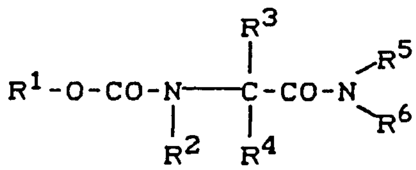

(I),

in welcher

$R^1$ für Alkyl, Alkenyl, Alkinyl, Halogenalkyl, Halogenalkenyl, Halogenalkinyl, Cycloalkyl, Cycloalkenyl, unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl steht,

$R^2$, $R^5$ gleich oder verschieden sind und für Wasserstoff oder $C_1$-$C_4$.-Alkyl stehen,

$R^3$, $R^4$ für Wasserstoff, Cycloalkyl oder Alkyl stehen oder

$R^3$ und $R^4$ zusammen mit dem Kohlenstoffatom, an das sie gebunden sind, einen Cycloalkylring bilden,

$R^6$ für unsubstituiertes oder substituiertes geradkettiges oder verzweigtes Cycloalkylalkyl, für unsubstituiertes oder substituiertes geradkettiges oder verzweigtes Cycloalkenylalkyl, durch Alkyl überbrücktes Cycloalkylalkyl steht,

ausgenommen die Verbindung N-(N-Benzyloxycarbonyl-glycyl)-aminomethylcyclohexan.

Die Verbindungen der Formel (I) können außerdem ein oder mehrere Chiralitätszentren enthalten und können somit in verschiedenen Enantiomeren- und Diastereomerengemischen vorliegen, die gegebenenfalls in üblicher Art und weise getrennt werden können. Sowohl die reinen Enantiomeren und Diastereomeren, als auch die Gemische werden erfindungsgemäß beansprucht.

Im folgenden wird der Einfachheit halber stets von Verbindungen der Formel (I) gesprochen, obwohl sowohl die reinen Verbindungen, als auch die Gemische mit unterschiedlichen Anteilen an isomeren, enantiomeren und diastereomeren Verbindungen gemeint sind.

Die erfindungsgemäß substituierten Aminosäureamid-Derivate sind durch die Formel (I) allgemein definiert.

Vorzugsweise bedeutet in den allgemeinen Formeln im folgenden, falls nicht anders definiert:

Alkyl, einzeln oder in zusammengesetzten Resten

- geradkettiges oder verzweigtes Alkyl mit 1 bis 6, insbesondere 1 bis 4 Kohlenstoffatomen. Beispielhaft und vorzugsweise seien Methyl, Ethyl, n.- und i.-Propyl, n-, i-, s- und t-Butyl, genannt.

Alkenyl

- geradkettiges oder verzweigtes Alkenyl mit 2 bis 6, insbesondere 2 bis 4 Kohlenstoffatomen. Beispielhaft und vorzugsweise seien gegebenenfalls substituiertes Ethenyl, Propenyl-(1), Propenyl-(2) und Butenyl-(3) genannt.

Alkinyl

- geradkettiges oder verzweigtes Alkinyl mit 2 bis 6, insbesondere 2 bis 4 Kohlenstoffatomen. Beispielhaft und vorzugsweise seien gegebenenfalls substituiertes Ethinyl, Propinyl-(1), Propinyl-(2) und Butinyl-(3) genannt.

Halogenalkyl

- geradkettiges oder verzweigtes Halogenalkyl mit 1 bis 6, insbesondere 1 bis 4 Kohlenstoffatomen und 1 bis 9, insbesondere 1 bis 5 und vor allem 1 bis 3, gleichen oder verschiedenen Halogenatomen wie Fluor, Chlor, Brom, Iod, insbesondere Fluor und Chlor. Beispielhaft und vorzugsweise seien Fluormethyl, Fluorethyl, Fluorpropyl, Chlorpropyl, Fluorbutyl, Chlorbutyl, Difluormethyl, Difluorethyl, Dichlorethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl und Trichlorbutyl genannt.

Halogenalkenyl und Halogenalkinyl

- geradkettiges oder verzweigtes gegebenenfalls substituiertes Halogenalkenyl bzw. Halogenalkinyl mit 2 bis 6, insbesondere 2 bis 4 Kohlenstoffatomen und 1 bis 9, insbesondere 1 bis 5 und vor allem 1 bis 3, gleichen oder verschiedenen Halogenatomen wie Fluor, Chlor, Brom, Iod, insbesondere Fluor und Chlor. Beispielhaft und vorzugsweise seien Fluorallyl, Chlorallyl, Fluorbutenyl, Chlorbutenyl, Fluorpropargyl, Chlorpropargyl, Fluorbutinyl und Chlorbutinyl genannt.

Cycloalkyl

- steht für einen 3- bis 7-gliedrigen substituierten oder unsubstituierten Ring, insbesondere für einen Ring mit 3, 5 oder 6 Kohlenstoffatomen. Beispielhaft und vorzugsweise seien Cyclopropyl, Cyclobutyl, Cyclopentyl, Cyclohexyl und Cycloheptyl genannt.

Cycloalkenyl

- substituiertes oder unsubstituiertes Cycloalkenyl mit 3 bis 7, insbesondere 3 bis 6 Kohlenstoffatomen und 1 oder 2 Doppelbindungen.

Aryl

- unsubstituiertes oder substituiertes Aryl mit 6 bis 10 Kohlenstoffatomen. Beispielhaft und vorzugsweise sei jeweils unsubstituiertes oder substituiertes Phenyl und Naphthyl, insbesondere unsubstituiertes oder substituiertes Phenyl genannt.

Aralkyl

- unsubstituiertes oder substituiertes Aralkyl mit 1 bis 4, insbesondere 1 oder 2 Kohlenstoffatomen im geradkettigen oder verzweigten Alkylteil und 6 bis 10 Kohlen-stoffatomen, vorzugsweise Phenyl im Arylteil. Beispielhaft und vorzugsweise seien Benzyl, 1,1- und 1,2-Phenethyl und 1,1-, 1,2-, 1,3- und 2,2-Phenylpropyl genannt.

Heteroaryl

- unsubstituierter oder substituierter 5- bis 9-gliedriger Ring, insbesondere 5- bis 7-gliedriger Ring, der 1 bis 4, bevorzugt 1 bis 3, gleiche oder verschiedene Heteroatome enthält. Als Heteroatome seien vorzugsweise Sauerstoff, Schwefel und Stickstoff genannt. Beispielhaft und vorzugsweise seien Pyrimidinyl, Thienyl, Furyl, Pyrazinyl, Thiazolyl und insbesondere Pyridyl genannt.

Heteroarylalkyl

- der Heteroarylteil entspricht den oben angegebenen Definitionen und Vorzugsbereichen. Der Alkylteil ist geradkettig oder verzweigt und enthält 1 bis 4 insbesondere 1 oder 2 Kohlenstoffatome. Beispielhaft und vorzugsweise seien Heteroarylmethyl, 1,1- und 1,2-Heteroarylethyl genannt.

Die gegebenenfalls substituierten Reste der allgemeinen Formeln können einen oder mehrere, vorzugsweise 1 bis 3, insbesondere 1 oder 2, gleiche oder verschiedene Substituenten tragen. Als Substituenten seien beispielhaft und vorzugsweise aufgeführt: Alkyl mit vorzugsweise 1 bis 4, insbesondere 1 oder 2 Kohlenstoffatomen, wie Methyl, Ethyl, n- und i-Propyl und n-, i-, sec- und t-Butyl; Alkoxy mit vorzugsweise 1 bis 4, insbesondere 1 oder 2 Kohlenstoffatomen, wie Methoxy, Ethoxy, n- und i.-Propyloxy und n-, i-, sec- und t-Butyloxy; Alkylthio mit vorzugsweise 1 bis 4, insbesondere 1 oder 2 Kohlenstoffatomen, wie Methylthio, Ethylthio, n- und i.-Propylthio und n-, i-, sec- und t-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Halogen, vorzugsweise Fluor, Chlor, Brom und Jod, insbesondere Fluor, Chlor und Brom; Alkylalkoxy mit 1 bis 4, insbesondere 1 oder 2 Kohlenstoffatomen in jedem Alkylteil; Carbonylalkoxy mit 1 bis 4, insbesondere 1 oder 2 Kohlenstoffatomen im Alkylteil, wie Carbonylmethoxy und Carbonylethoxy.

Die hier aufgeführten Definitionen gelten in entsprechender Weise auch für die Definitionen in den folgenden bevorzugten Kombinationen von Resten.

Bevorzugt sind die Verbindungen der Formel (I), in welcher

$R^1$ für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweigtes Halogenalkenyl oder Halogenalkinyl mit 2 bis 6 Kohlenstoffatomen und 1 bis 9 gleichen oder verschiedenen Halogenatomen oder substituiertes oder unsubstituiertes Cycloalkyl oder Cycloalkenyl mit 3 bis 7 Kohlenstoffatomen steht, für jeweils unsubstituiertes oder substituiertes Phenyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Pyridyl- bzw. Phenylteil jeweils in Frage kommen:
Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Halogen; Alkylalkoxy mit 1 bis 4 Kohlenstoffatomen in jedem Alkylteil; Carbonylalkoxy mit 1 bis 4 Kohlenstoffatomen im Alkylteil;

$R^2$ und $R^5$ für Wasserstoff oder Methyl stehen,

$R^3$ und $R^4$ gleich oder verschieden sind und für Wasserstoff, unsubstituiertes oder substituiertes Cycloalkyl mit 3 bis 7 Kohlenstoffatomen oder geradkettiges oder verzweigtes Alkyl mit 1 bis 6 Kohlenstoffatomen stehen oder

$R^3$ und $R^4$ zusammen mit dem Kohlenstoffatom, an das sie gebunden sind, einen Cycloalkylring mit 3 bis 7 Kohlenstoffatomen bilden und

$R^6$ für unsubstituiertes oder substituiertes Cycloalkylalkyl mit 3 bis 7 Kohlenstoffatomen im Cycloalkylteil und 1 bis 6 Kohlenstoffatomen im geradkettigen oder verzweigten Alkylteil steht, für unsubstituiertes oder substituiertes Cycloalkenylalkyl mit 5 bis 8 Kohlenstoffatomen im Cycloalkenylteil und 1 bis 6 Kohlenstoffatomen im geradkettigen oder verzweigten Alkylteil steht, für unsubstituiertes oder substituiertes, durch Alkyl überbrücktes Cycloalkylalkyl mit 5 bis 8 Kohlenstoffatomen im Cycloalkylteil, mit 1 bis 4 Kohlenstoffatomen im verbrückenden Alkylteil und 1 bis 6 Kohlenstoffatomen im geradkettigen oder verzweigten Alkylteil steht, wobei als Substituenten in Frage kommen:
Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Dialkylamino mit 1 bis 4 Kohlenstoffatomen je Alkylgruppe; Alkylalkoxy mit 1 bis 4 Kohlenstoffatomen in jedem Alkylteil.

Besonders bevorzugt sind diejenigen Verbindungen der Formel (I), in denen

$R^1$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und weiterhin für unsubstituiertes oder einfach bis dreifach, gleich oder verschieden substituiertes Phenyl steht, wobei die folgenden Substituenten in Frage kommen:
Alkyl, Alkoxy und Alkylthio mit jeweils 1 bis 4 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Fluor, Chlor, Brom und Jod; Alkylalkoxy mit 1 bis 2 Kohlenstoffatomen in jedem Alkylteil; Carbonylalkoxy mit 1 oder 2 Kohlenstoffatomen im Alkylteil;

$R^2$ und $R^5$ für Wasserstoff stehen,

$R^3$ und $R^4$ gleich oder verschieden sind und für Wasserstoff, unsubstituiertes oder substituiertes Cycloalkyl mit 3 bis 6 Kohlenstoffatomen oder geradkettiges oder verzweigtes Alkyl mit 1 bis 5 Kohlenstoffatomen stehen oder

$R^3$ und $R^4$ zusammen mit dem Kohlenstoffatom, an das sie gebunden sind, einen Cycloalkylring mit 3 bis 6 Kohlenstoffatomen bilden und

$R^6$ für unsubstituiertes oder substituiertes Cycloalkylalkyl mit 3 bis 6 Kohlenstoffatomen im Cycloalkylteil und 1 bis 4 Kohlenstoffatomen im geradkettigen oder verzweigten Alkylteil steht, für unsubstituiertes oder substituiertes Cycloalkenylalkyl mit 5 bis 7 Kohlenstoffatomen im Cycloalkenylteil und 1 bis 4 Kohlenstoffatomen im geradkettigen oder verzweigten Alkylteil steht, für unsubstituiertes oder substituiertes, durch Alkyl überbrücktes Cycloalkylalkyl mit 5 bis 7 Kohlenstoffatomen im Cycloalkylteil, mit 1 bis 3 Kohlenstoffatomen im verbrückenden Alkylteil und 1 bis 4 Kohlenstoffatomen im geradkettigen oder verzweigten Alkylteil steht, wobei als Substituenten in Frage kommen: Alkyl, Alkoxy und Alkylthio mit jeweils 1 bis 4 Kohlenstoffatomen; Halogenalkyl, Halogenalkoxy und Halogenalkylthio mit 1 bis 2 Kohlenstoffatomen und 1 bis 5 Halogenatomen, wobei die Halogenatome gleich oder verschieden sind; Hydroxy; Dialkylamino mit 1 bis 2 Kohlenstoffatomen je Alkylgruppe; Alkylalkoxy mit 1 bis 2 Kohlenstoffatomen in jedem Alkylteil.

Ganz besonders bevorzugt werden diejenigen Verbindungen der Formel (I), in denen

$R^1$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor- oder Chlorallyl, -butenyl, -propargyl, -butinyl, Cyclopropyl, Cyclopentyl, Cyclohexyl, Cyclopentenyl oder Cyclohexenyl, für unsubstituiertes oder einfach bis zweifach, gleich oder verschieden substituiertes Phenyl steht, wobei die folgenden Substituenten in Frage kommen: Methyl, Ethyl, n- und i-Propyl, n-, i-, s-und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, und

$R^2$ und $R^5$ für Wasserstoff stehen und

$R^3$ und $R^4$ gleich oder verschieden sind und für Wasserstoff, Methyl, Ethyl, n- oder i-Propyl, n-, i-, s- oder t-Butyl, 3-Pentyl, Cyclopropyl, Cyclopentyl oder Cyclohexyl stehen oder

$R^3$ und $R^4$ zusammen mit dem Kohlenstoffatom, an das sie gebunden sind, einen Cyclopropyl-, Cyclopentyl- oder Cyclohexylring bilden oder insbesondere

$R^3$ für i-Propyl, s-Butyl oder Cyclopentyl und

$R^4$ für Wasserstoff steht,

$R^6$ für unsubstituiertes oder substituiertes Cycloalkylalkyl mit 3, 5 oder 6 Kohlenstoffatomen im Cycloalkylteil und 1 bis 3 Kohlenstoffatomen im geradkettigen oder verzweigten Alkylteil steht, für unsubstituiertes oder substituiertes Cycloalkenylalkyl mit 5 bis 7 Kohlenstoffatomen im Cycloalkenylteil und 1 bis 3 Kohlenstoffatomen im geradkettigen oder verzweigten Alkylteil steht, für unsubstituiertes oder substituiertes, durch Alkyl überbrücktes Cycloalkylalkyl mit 5 bis 7 Kohlenstoffatomen im Cycloalkylteil, mit 1 bis 2 Kohlenstoffatomen im verbrückenden Alkylteil und 1 bis 3 Kohlenstoffatomen im geradkettigen oder verzweigten Alkylteil steht, wobei als Substituenten in Frage kommen:
Methyl, Ethyl, n- und i-Propyl, n-, i-, s- und t-Butyl, Methoxy, Ethoxy, i- und n- Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio, Hydroxy, Methylthio, Ethylthio, n- und i-Propylthio, Dimethylamino, Diethylamino.

Man erhält die substituierten Aminosäureamid-Derivate der allgemeinen Formel (I)

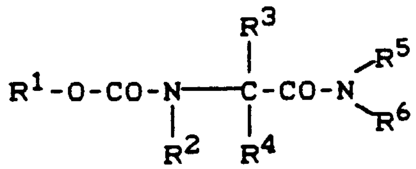 (I),

in welcher

$R^1$, $R^2$, $R^3$, $R^4$, $R^5$ und $R^6$ die oben angegebene Bedeutung haben,

wenn man

A) eine substituierte Aminosäure der Formel (II)

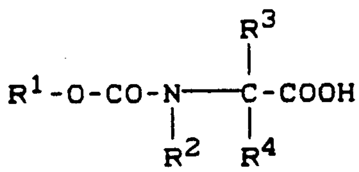 (II),

in welcher

$R^1$, $R^2$, $R^3$ und $R^4$ die oben angegebene Bedeutung haben, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels

mit einem Amin der Formel (III)

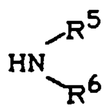 (III),

in welcher

$R^5$ und $R^6$ die oben angegebene Bedeutung haben, umsetzt, oder

B) substituierte Aminosäureamid-Derivate der Formel (IV)

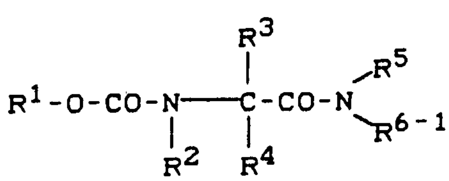 (IV),

in welcher

$R^1$, $R^2$, $R^3$, $R^4$ und $R^5$ die oben angegebene Bedeutung haben und

$R^{6-1}$ für unsubstituiertes oder substituiertes Phenyl steht, wobei als Phenylsubstituenten die für $R^6$ oben genannten Substituenten in Frage kommen,

in Gegenwart eines Katalysators, gegebenenfalls in Gegenwart eines Inertgases und in Gegenwart von Wasserstoff, gegebenenfalls in Gegenwart eines Verdünnungsmittels und gegebenenfalls unter Druck hydriert.

Verwendet man beispielsweise tert.-Butoxycarbonyl-D/L-Valin und 1-Cyclohexylethylamin so kann der Ablauf des erfindungsgemäßen Verfahrens A durch das folgende Formelschema wiedergegeben werden:

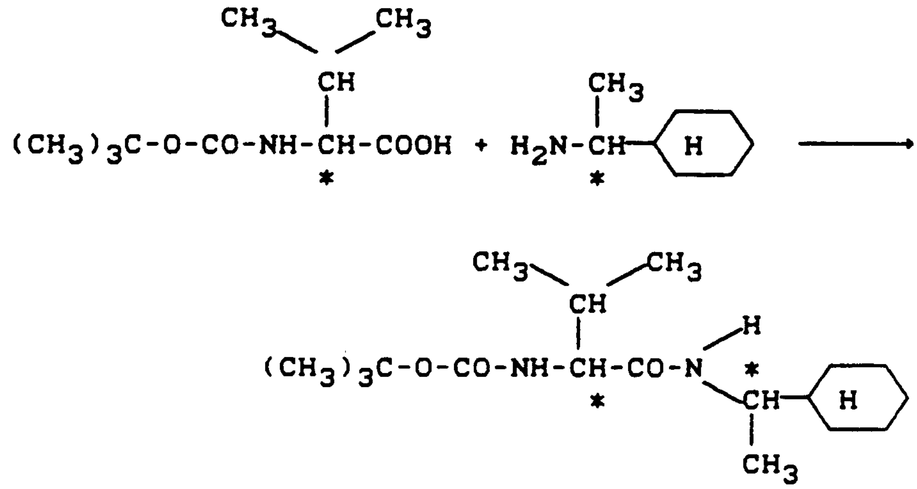

Verwendet man beispielsweise N-(i-Propyloxy-carbonyl)-L-valin-phenylethylamid, Rhodium als Katalysator und Wasserstoff, so kann der Ablauf des erfindungsgemäßen Verfahrens B durch das folgende Formelschema wiedergegeben werden:

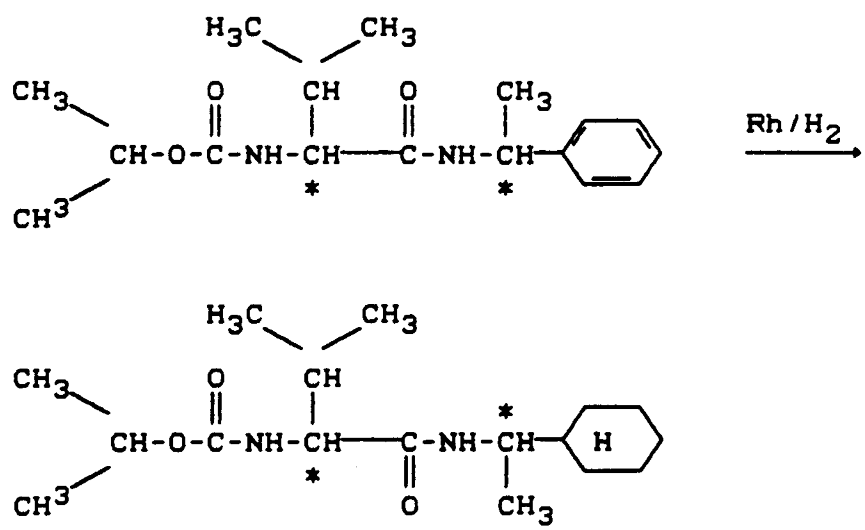


Die für die Durchführung des erfindungsgemäßen Verfahrens (A) als Ausgangsstoffe zu verwendenden Aminosäure-Derivate sind durch die Formel (II) allgemein definiert. In dieser Formel haben $R^1$, $R^2$, $R^3$ und $R^4$ vorzugsweise die Bedeutungen, die bereits im Zusammenhang mit der Beschreibung der erfindungsgemäßen Stoffe der Formel (I) für diese Substituenten bevorzugt genannt wurden.

Die Aminosäure-Derivate der Formel (II) sind allgemein bekannt (vgl. z.B. Houben-Weyl, Methoden der organischen Chemie, Band XV, Teil 1 und 2, Seiten 46 ff und 112 ff, Georg Thieme Verlag, Stuttgart 1974; D. Keller et al., Org. Synth. 60, 2145 (1981); bzw. R.C. Sheppard, A Specialist Periodical Report, Amino-acids, Peptides and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965) oder können nach den dort angegebenen Verfahren erhalten werden.

Die außerdem für die Durchführung des erfindungsgemäßen Verfahrens als Ausgangsstoffe zu verwendenden carboxy-aktivierten Derivate der Aminosäure der Formel (II) sind allgemein bekannt.

Als carboxy-aktivierte Derivate der Aminosäuren der Formel (II) kommen alle Carboxy-aktivierten Derivate in Frage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxysuccinimidester sowie mit Kondensationsmitteln, wie z.B. Dicyclohexylcarbodiimid oder Carbonyldiimidazol, in situ erzeugte aktivierte Formen der Aminosäuren.

Vorzugsweise werden die den Aminosäuren der Formel (II) entsprechenden Säurechloride und gemischten Anhydride eingesetzt. Sie können hergestellt werden, indem man die Aminosäuren der Formel (II) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt. Bevorzugt ist der Einsatz von Chlorameisensäureisobutylester.

Die Umsetzung kann in Gegenwart indifferenter Verdünnungsmittel wie z.B. aromatische, nichtaromatische oder halogenierte Kohlenwasserstoffe wie: Ketone, wie z.B. Aceton; Ester, wie z.B. Ethylacetat; Amide, wie z.B. Dimethylformamid; Nitrile, wie z.B. Acetonitril, Chlorkohlenwasserstoffe, wie z.B. Methylenchlorid, Kohlenwasserstoffe, wie z.B. Toluol; oder Ether, wie z.B. Tetrahydrofuran bzw. deren Mischungen und/oder in Gegenwart eines Säurebindemittels, wie vorzugsweise eines tertiären Amins, wie z.B. Triethylamin, Pyridin oder N-Methylpiperidin, bei Temperaturen von -78 °C bis 100 °C, vorzugsweise -60 °C bis 25 °C, durchgeführt werden.

Die außerdem für die Durchführung des erfindungsgemäßen Verfahrens als Ausgangsstoffe zu verwendenden Amine sind durch die Formel (III) allgemein definiert. In diesen Formeln haben $R^1$, Ar', $R^5$ und $R^6$ die oben genannten Bedeutungen.

Die Amine der Formel (III) sind allgemein bekannte Verbindungen der organischen Chemie.

Als Verdünnungsmittel kommen für das erfindungsgemäße Verfahren (A) inerte, organische Lösungsmittel wie: Ketone, wie Aceton oder Ethylmethylketon; Ester wie Ethyl- oder Methylacetat; Amide wie Dimethylformamid; Nitrile, wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren übliche anorganische und organische Säurebinder in Frage. Hierzu gehören vorzugsweise tertiäre Amine, wie Triethylamin, Pyridin oder N-Methylpiperidin, sowie anorganische Basen, z.B. Metallhydroxide wie Natrium- und Kaliumhydroxid oder Metallcarbonate wie Natriumcarbonat oder Calciumcarbonat.

Das erfindungsgemäße Verfahren (A) wird gegebenenfalls in Gegenwart eines Katalysators durchgeführt. Beispielsweise genannt seien 4-Dimethylaminopyridin, 1-Hydroxybenzotriazol oder Dimethylformamid.

Die Temperaturen können bei der Durchführung des Verfahrens (A) in einem größeren Bereich variiert werden. Im allgemeinen arbeitet man bei -78 bis +120 °C, vorzugsweise bei -60 bis +40 °C.

Bei der Durchführung des erfindungsgemäßen Verfahrens (A) arbeitet man vorzugsweise in äquimolaren Mengen.

Dabei werden die Aminosäurederivate der Formel (II) als reine optische Isomere (D bzw. L-Form) oder als Racemate eingesetzt.

Die für die Durchführung des erfindungsgemäßen Verfahrens (B) als Ausgangsstoffe zu verwendeten Aminosäureamid-Derivate sind durch die Formel (IV) allgemein definiert. In dieser Formel haben $R^1$, $R^2$, $R^3$, $R^4$, $R^5$ und $R^{6-1}$ vorzugsweise die Bedeutungen, die bereits im Zusamenhang mit der Beschreibung der erfindungsgemäßen Stoffe der Formel (I) für diese Substituenten genannt wurden.

Die substituierten Aminosäureamid-Derivate der Formel (IV) sind neu und Gegenstand einer nicht zum veröffentlichen Stand der Technik gehörenden Patentanmeldung der Anmelderin (vgl. die Deutschen Patentanmeldungen P 4 026 966, P 3 936 298 und P 3 915 755).

Man erhält die neuen Aminosäureamid-Derivate der Formel (IV) beispielsweise, in dem man substituierte Aminosäuren der Formel (V)

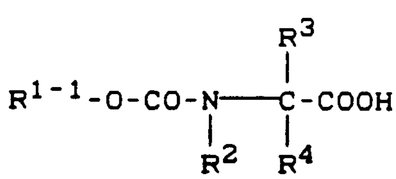

(V),

in welcher

$R^2$, $R^3$ und $R^4$ die oben angegebene Bedeutung haben und

$R^{1-1}$ für Alkyl und Cycloalkyl steht, bzw. deren carboxyaktivierte Derivate
mit Aminen der Formel (VI)

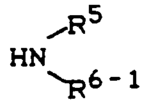 (VI),

in welcher

$R^5$ die oben angegebene Bedeutung hat und

$R^{6-1}$ für unsubstituiertes oder substituiertes Phenyl steht, wobei als Phenylsubstituenten die für $R^6$ oben genannten Substituenten in Frage kommen in Dichlormethan, in Gegenwart von beispielsweise N-Methylpiperidin als Säurebindemittel und Chlorameisensäureisobutylester bei Temperaturen zwischen -60 °C und -10 °C umsetzt.

Die Ameisensäuren der Formel (V) und die Amine der Formel (IV) sind allgemein bekannte Verbindungen der organischen Chemie.

Als Verdünnungsmittel kommen für das erfindungsgemäße Verfahren B inerte, organische Lösungsmittel in Frage, beispielweise Alkohole, wie Ethanol, Isopropanol oder s-Butanol; gesättigte Kohlenwasserstoffe wie n-Hexan oder Cyclohexan.

Die Temperaturen können bei der Durchführung des erfindungsgemäßen Verfahren (B) in einem größeren Bereich variiert werden. Im allgemeinen arbeitet man bei 50 bis 250 °C, vorzugsweise bei 0 bis 200 °C.

Das Verfahren (B) zur Herstellung der neuen Aminosäureamid-Derivate der Formel (I) wird im allgemeinen unter erhöhtem Druck durchgeführt. Im allgemeinen arbeitet man bei einem Druck von 50 bis 250 bar, vorzugsweise bei einem Druck von 100 bis 200 bar.

Unter bestimmten Voraussetzungen kann jedoch auch unter Normaldruck oder vermindertem Druck gearbeitet werden.

Als Inertgase kommen dabei Stickstoff sowie praktisch alle Edelgase, insbesondere Argon in Frage.

Zur Durchführung des erfindungsgemäßen Verfahrens (B) setzt man auf 1 Mol des substituierten Aminosäureamid-Derivats der Formel (IV) im allgemeinen 0,001 bis 1 Mol, vorzugsweise 0,005 bis 0,5 Mol Katalysator ein.

Das erfindungsgemäße Verfahren (B) wird mit den für derartige Reaktionen üblichen Katalysatoren durchgeführt. Vorzugsweise können hierfür die Metalle und/oder Salze oder Komplexe von Metallen der VIII. Nebengruppe des Periodensystems der Elemente verwendet werden; insbesondere seien Ruthenium, Rhodium, Palladium und Platin genannt.

Die Erfindung umfaßt sowohl die reinen Isomeren als auch die Gemische. Diese Gemische können nach gebräuchlichen Methoden, z.B. selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so z.B. durch Salzbildung mit optisch aktiven Säuren wie Champhersulfonsäure oder Dibenzoylweinsäure und selektive Kristallisation oder durch Derivatisierung mit geeigneten, optisch aktiven Reagenzien, Trennung der diastereomeren Derivate und Rückspaltung oder Trennung an optisch aktivem Säulenmaterial.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Phytophthora-Arten an Tomaten oder Plasmopara-Arten an Reben einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächen-aktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut von Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

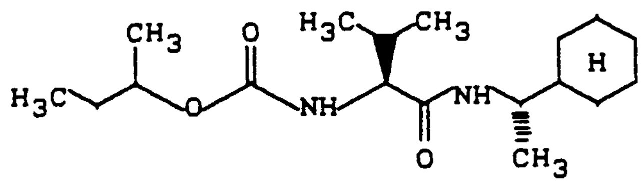

In einem Autoklaven werden 3,2 g (10 mmol) $N^2$-sec.-Butyloxycarbonyl-$N^1$-(S(-)-1-phenylethyl)-L-valinamid in 50 ml sec-Butanol gelöst und mit 0,5 g Rhodium-schwarz als Katalysator versetzt. Man hydriert die Mischung 12 Stunden lang bei einem Wasserstoffdruck von 150 bar und einer Temperatur von 150 °C. Zur Aufarbeitung wird vom Katalysator abfiltriert und das Filtrat im Vakuum vom Lösungsmittel befreit. Der Rückstand wird zur Reinigung einmal mit Diisopropyl-ether verrührt. Man erhält 2,93 g (90 % d.Th.) farbloses $N^2$-sec.-Butyloxycarbonyl-$N^1$-(S(+)-1-cyclohexylethyl)-L-valinamid mit einem Schmelzpunkt von 186 °C.

Analog Beispiel 1 werden die folgenden Verbindungen der Formel (I) erhalten:

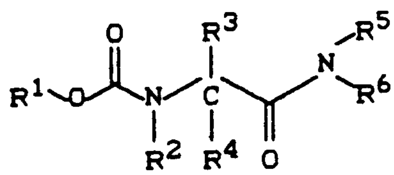

Tabelle 1

| Nr. | $R^1$ | $R^2$ | $R^3$ | $R^4$ | $R^5$ | $R^6$ | phys.Konstante |
|---|---|---|---|---|---|---|---|
| 2 | $-CH(CH_3)-CH_2CH_3$ | H | $-CH(CH_3)_2$ | H | H | $-CH(CH_3)-$ 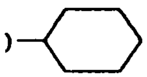 | mp.: 186° C |
| 3 | 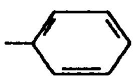 | H | $-CH(CH_3)_2$ | H | H | $-CH(CH_3)-$ 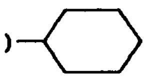 | mp.: 146-147° C |
| 4 | $-CH(CH_3)_2$ | H | $-CH(CH_3)_2$ | H | H | $-CH(CH_3)-$ 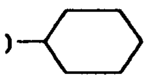 | mp.: 190° C |
| 5 | $-CH(CH_3)-CH_2CH_3$ | H | $-CH(CH_3)_2$ | H | H | $-CH(CH_3)-$ 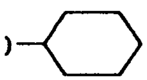 | mp.: 178° C<br>(R-)-Amin |
| 6 | $-CH(CH_3)_2$ | H | $-CH(CH_3)_2$ | H | H | $-CH(CH_3)-$ 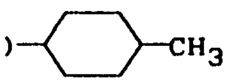 $CH_3$ | mp.: 111° C |

**Tabelle 1** Fortsetzung

| Nr. | $R^1$ | $R^2$ | $R^3$ | $R^4$ | $R^5$ | $R^6$ | phys.Konstante |
|---|---|---|---|---|---|---|---|
| 7 | $-CH(CH_3)_2$ | H | $-CH(CH_3)_2$ | H | H | $-CH(CH_3)-$ 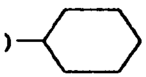 | mp.: 165° C<br>(R-)-Amin |
| 8 | $-CH(CH_3)_2$ | H | $-CH(CH_3)_2$ | H | H | $-CH(CH_3)-$ 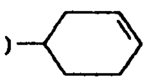 | mp.: 145° C |
| 9 | $-CH(CH_3)_2$ | H | $-CH(CH_3)_2$ | H | H | $-CH(CH_3)$ 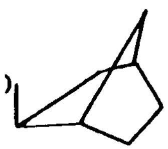 | mp.: 123° C |
| 10 | $-CH(CH_3)-CH_2CH_3$ | H | $-CH(CH_3)_2$ | H | H | $-CH(CH_3)-$ 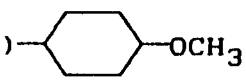 | mp.: 125° C |
| 11 | $-CH(CH_3)_2$ | H | $-CH(CH_3)_2$ | H | H | $-CH(CH_3)-$ 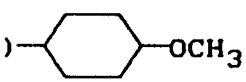 | mp.: 127° C |

**Tabelle 1** Fortsetzung

| Nr. | $R^1$ | $R^2$ | $R^3$ | $R^4$ | $R^5$ | $R^6$ | phys.Konstante |
|---|---|---|---|---|---|---|---|
| 12 | $-CH(CH_3)-CH_2CH_3$ | H | $-CH(CH_3)_2$ | H | H | $-CH(CH_3)$ 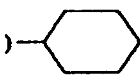 $CH_3$ | mp.: 100° C |
| 13 | $-CH(CH_3)_2$ | H | $-CH(CH_3)_2$ | H | H | $-CH(CH_3)$ 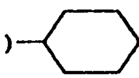 $CH_2-CH_3$ | mp.: 150° C |
| 14 | $-CH(CH_3)_2$ | H | $-CH(CH_3)_2$ | H | H | $-CH(CH_3)$ 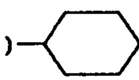 $CH_3$ | mp.: 153° C (R+)-Amin |
| 15 | $-CH(CH_3)_2$ | H | $-CH(CH_3)_2$ | H | H | $-CH(CH_3)$ 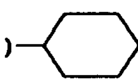 $CH_2-CH_3$ | mp.: 118° C (R+)-Amid |
| 16 | $-CH(CH_3)CH_2CH_3$ | H | $-CH(CH_3)_2$ | H | H | $-CH(CH_3)$ 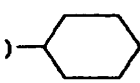 $CH_3$ | mp.: 90° C (R+)-Amid |

Tabelle 1 Fortsetzung

| Nr. | $R^1$ | $R^2$ | $R^3$ | $R^4$ | $R^5$ | $R^6$ | phys.Konstante |
|---|---|---|---|---|---|---|---|
| 17 | $-CH(CH_3)_2$ | H | $-CH(CH_3)_2$ | H | H | $-CH(CH_3)-$ 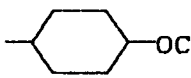 $-OCH_3$ | mp.: 115° C (R+)-Amid |
| 18 | $-CH(CH_3)-CH_2CH_3$ | H | $-CH(CH_3)_2$ | H | H | $-CH(CH_3)-$ 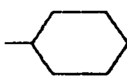 $-OCH_3$ | Racemat |
| 19 | $-CH(CH_3)-CH_2CH_3$ | H | $-CH(CH_3)_2$ | H | H | $-CH(CH_3)-$ 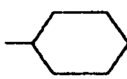 $-CH_2CH_3$ | Racemat (R+)-Amin |
| 20 | $-CH(CH_3)-CH_2CH_3$ | H | $-CH(CH_3)_2$ | H | H | $-CH(CH_3)-$ 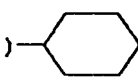 $-CH_2CH_3$ | (R+)-Amid |

Beispiel A

Phytophthora-Test (Tomate) /protektiv

Lösungsmittel: 4,7 Gewichtsteile Aceton
Emulgator: 0,3 Gewichtsteile Alkyl-Aryl-Polyglykolkolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in eienr Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 6, 7, 12, 13, 14

**Patentansprüche**

**1.** Aminosäureamid-Derivate der allgemeinen Formel (I)

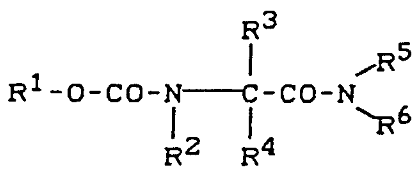

(I),

in welcher

| | |
|---|---|
| $R^1$ | für Alkyl, Alkenyl, Alkinyl, Halogenalkyl, Halogenalkenyl, Halogenalkinyl, Cycloalkyl, Cycloalkenyl, unsubstituiertes oder substituiertes Aryl, unsubstituiertes oder substituiertes Aralkyl, unsubstituiertes oder substituiertes Heteroaryl und unsubstituiertes oder substituiertes Heteroarylalkyl steht, |
| $R^2$, $R^5$ | gleich oder verschieden sind und für Wasserstoff oder $C_1$-$C_4$-Alkyl stehen, |
| $R^3$, $R^4$ | für Wasserstoff, Cycloalkyl oder Alkyl stehen oder |
| $R^3$ und $R^4$ | zusammen mit dem Kohlenstoffatom, an das sie gebunden sind, einen Cycloalkylring bilden, |
| $R^6$ | für unsubstituiertes oder substituiertes geradkettiges oder verzweigtes Cycloalkylalkyl, für unsubstituiertes oder substituiertes geradkettiges oder verzweigtes Cycloalkenylalkyl, durch Alkyl überbrücktes Cycloalkylalkyl steht,ausgenommen die Verbindung N-(N-Benzyloxycarbonyl-glycyl)-aminomethylcyclohexan |

**2.** Verbindungen der Formel (I), gemäß Anspruch 1
in welcher

| | |
|---|---|
| $R^1$ | für geradkettiges oder verzweigtes Alkyl mit 1 bis 6 Kohlenstoffatomen, geradkettiges oder verzweigtes Alkenyl oder Alkinyl mit 2 bis 6 Kohlenstoffatomen, geradkettiges oder verzweigtes Halogenalkyl mit 1 bis 6 Kohlenstoffatomen und 1 bis 9 gleichen oder verschiedenen Halogenatomen, geradkettiges oder verzweigtes Halogenalkenyl oder Halogenalkinyl mit 2 bis 6 Kohlenstoffatomen und 1 bis 9 gleichen oder verschiedenen Halogenatomen oder substituiertes oder unsubstituiertes Cycloalkyl oder Cycloalkenyl mit 3 bis 7 Kohlenstoffatomen steht, für jeweils unsubstituiertes oder substituiertes Phenyl und Pyridyl oder unsubstituiertes oder im Phenylteil substituiertes Phenylalkyl mit 1 bis 4 Kohlenstoffatomen im geradkettigen oder verzweigten Alkylteil stehen, wobei als Substituenten im Pyridyl- bzw. Phenylteil jeweils in Frage kommen: |
| | Alkyl, Alkoxy und Alkylthio mit jeweils 1 bis 4 Kohlenstoffatomen; Halogenalkyl, |

Halogenalkoxy und Halogenalkylthio mit jeweils 1 bis 4 Kohlenstoffatomen und 1 bis 9 Halogenatomen, wobei die Halogenatome gleich oder verschieden sind; Halogen; Alkylalkoxy mit 1 bis 4 Kohlenstoffatomen in jedem Alkylteil; Carbonylalkoxy mit 1 bis 4 Kohlenstoffatomen im Alkylteil;

$R^2$ und $R^5$ für Wasserstoff oder Methyl stehen,

$R^3$ und $R^4$ gleich oder verschieden sind und für Wasserstoff, unsubstituiertes oder substituiertes Cycloalkyl mit 3 bis 7 Kohlenstoffatomen oder geradkettiges oder verzweigtes Alkyl mit 1 bis 6 Kohlenstoffatomen stehen oder

$R^3$ und $R^4$ zusammen mit dem Kohlenstoffatom, an das sie gebunden sind, einen Cycloalkylring mit 3 bis 7 Kohlenstoffatomen bilden und

$R^6$ für unsubstituiertes oder substituiertes Cycloalkylalkyl mit 3 bis 7 Kohlenstoffatomen im Cycloalkylteil und 1 bis 6 Kohlenstoffatomen im geradkettigen oder verzweigten Alkylteil steht, für unsubstituiertes oder substituiertes Cycloalkenylalkyl mit 5 bis 8 Kohlenstoffatomen im Cycloalkenylteil und 1 bis 6 Kohlenstoffatomen im geradkettigen oder verzweigten Alkylteil steht, für unsubstituiertes oder substituiertes, durch Alkyl überbrücktes Cycloalkylalkyl mit 5 bis 8 Kohlenstoffatomen im Cycloalkylteil, mit 1 bis 4 Kohlenstoffatomen im verbrückenden Alkylteil und 1 bis 6 Kohlenstoffatomen im geradkettigen oder verzweigten Alkylteil steht, wobei als Substituenten in Frage kommen:

Alkyl, Alkoxy und Alkylthio mit jeweils 1 bis 4 Kohlenstoffatomen; Halogenalkyl, Halogenalkoxy und Halogenalkylthio mit jeweils 1 bis 4 Kohlenstoffatomen und 1 bis 9 Halogenatomen, wobei die Halogenatome gleich oder verschieden sind; Hydroxy; Dialkylamino mit 1 bis 4 Kohlenstoffatomen je Alkylgruppe; Alkylalkoxy mit 1 bis 4 Kohlenstoffatomen in jedem Alkylteil.

**3.** Verbindungen der Formel (I), gemäß Anspruch 1 in denen

$R^1$ für geradkettiges oder verzweigtes Alkyl mit 1 bis 4 Kohlenstoffatomen, geradkettiges oder verzweigtes Alkenyl oder Alkinyl mit 2 bis 4 Kohlenstoffatomen, geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl oder Halogenalkinyl mit 2 bis 4 Kohlenstoffatomen und 1 bis 5 gleichen oder verschiedenen Halogenatomen oder unsubstituiertes oder substituiertes Cycloalkyl oder Cycloalkenyl mit 3 bis 6 Kohlenstoffatomen steht und weiterhin für unsubstituiertes oder einfach bis dreifach, gleich oder verschieden substituiertes Phenyl steht, wobei die folgenden Substituenten in Frage kommen:

Alkyl, Alkoxy und Alkylthio mit jeweils 1 bis 4 Kohlenstoffatomen; Halogenalkyl, Halogenalkoxy und Halogenalkylthio mit 1 oder 2 Kohlenstoffatomen und 1 bis 5 Halogenatomen, wobei die Halogenatome gleich oder verschieden sind; Fluor, Chlor, Brom und Jod; Alkylalkoxy mit 1 bis 2 Kohlenstoffatomen in jedem Alkylteil; Carbonylalkoxy mit 1 oder 2 Kohlenstoffatomen im Alkylteil;

$R^2$ und $R^5$ für Wasserstoff stehen,

$R^3$ und $R^4$ gleich oder verschieden sind und für Wasserstoff, unsubstituiertes oder substituiertes Cycloalkyl mit 3 bis 6 Kohlenstoffatomen oder geradkettiges oder verzweigtes Alkyl mit 1 bis 5 Kohlenstoffatomen stehen oder

$R^3$ und $R^4$ zusammen mit dem Kohlenstoffatom, an das sie gebunden sind, einen Cycloalkylring mit 3 bis 6 Kohlenstoffatomen bilden und

$R^6$ für unsubstituiertes oder substituiertes Cycloalkylalkyl mit 3 bis 6 Kohlenstoffatomen im Cycloalkylteil und 1 bis 4 Kohlenstoffatomen im geradkettigen oder verzweigten Alkylteil steht, für unsubstituiertes oder substituiertes Cycloalkenylalkyl mit 5 bis 7 Kohlenstoffatomen im Cycloalkenylteil und 1 bis 4 Kohlenstoffatomen im geradkettigen oder verzweigten Alkylteil steht, für unsubstituiertes oder substituiertes, durch Alkyl überbrücktes Cycloalkylalkyl mit 5 bis 7 Kohlenstoffatomen im Cycloalkylteil, mit 1 bis 3 Kohlenstoffatomen im verbrückenden Alkylteil und 1 bis 4 Kohlenstoffatomen im geradkettigen oder verzweigten Alkylteil steht, wobei als Substituenten in Frage kommen:

Alkyl, Alkoxy und Alkylthio mit jeweils 1 bis 4 Kohlenstoffatomen; Halogenalkyl, Halogenalkoxy und Halogenalkylthio mit 1 bis 2 Kohlenstoffatomen und 1 bis 5

Halogenatomen, wobei die Halogenatome gleich oder verschieden sind; Hydroxy; Dialkylamino mit 1 bis 2 Kohlenstoffatomen je Alkylgruppe; Alkylalkoxy mit 1 bis 2 Kohlenstoffatomen in jedem Alkylteil.

**4.** Verbindungen der Formel (I), gemäß Anspruch 1
in denen

$R^1$ für Methyl, Ethyl, n- und i-Propyl, n-, i-, s- und t-Butyl, Fluormethyl, Fluorethyl, Fluorpropyl, Chlorpropyl, Fluorbutyl, Chlorbutyl, Difluormethyl, Difluorpropyl, Dichlorpropyl, Difluorbutyl, Dichlorbutyl, Trifluormethyl, Trichlormethyl, Trifluorethyl, Trichlorethyl, Trifluorpropyl, Trichlorpropyl, Trifluorbutyl, Trichlorbutyl, Allyl, Butenyl, Propargyl, Butinyl, Fluor-oder Chlor-allyl, -butenyl, -propargyl, -butinyl, Cyclopropyl, Cyclopentyl, Cyclohexyl, Cyclopentenyl oder Cyclohexenyl, für unsubstituiertes oder einfach bis zweifach, gleich oder verschieden substituiertes Phenyl steht, wobei die folgenden Substituenten in Frage kommen:
Methyl, Ethyl, n- und i-Propyl, n-, i-, s-und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio; Chlor, Brom, Fluor, und

$R^2$ und $R^5$ für Wasserstoff stehen und

$R^3$ und $R^4$ gleich oder verschieden sind und für Wasserstoff, Methyl, Ethyl, n- oder i-Propyl, n-, i-, s-oder t-Butyl, 3-Pentyl, Cyclopropyl, Cyclopentyl oder Cyclohexyl stehen oder

$R^3$ und $R^4$ zusammen mit dem Kohlenstoffatom, an das sie gebunden sind, einen Cyclopropyl-, Cyclopentyl-oder Cyclohexylring bilden,

$R^6$ für unsubstituiertes oder substituiertes Cycloalkylalkyl mit 3, 5 oder 6 Kohlenstoffatomen im Cycloalkylteil und 1 bis 3 Kohlenstoffatomen im geradkettigen oder verzweigten Alkylteil steht, für unsubstituiertes oder substituiertes Cycloalkenylalkyl mit 5 bis 7 Kohlenstoffatomen im Cycloalkenylteil und 1 bis 3 Kohlenstoffatomen im geradkettigen oder verzweigten Alkylteil steht, für unsubstituiertes oder substituiertes, durch Alkyl überbrücktes Cycloalkylalkyl mit 5 bis 7 Kohlenstoffatomen im Cycloalkylteil, mit 1 bis 2 Kohlenstoffatomen im verbrückenden Alkylteil und 1 bis 3 Kohlenstoffatomen im geradkettigen oder verzweigten Alkylteil steht, wobei als Substituenten in Frage kommen:
Methyl, Ethyl, n- und i-Propyl, n-, i-, s- und t-Butyl, Methoxy, Ethoxy, i- und n-Propoxy, n-, i-, s- und t-Butoxy, Trifluormethyl, Difluormethyl, Pentafluorethyl, Tetrafluorethyl, Trifluorchlorethyl, Trifluorethyl, Trifluorethoxy, Difluormethoxy, Pentafluorethoxy, Tetrafluorethoxy, Trifluorchlorethoxy, Trifluormethoxy und Trifluormethylthio, Hydroxy, Methylthio, Ethylthio, n- und i-Propylthio, Dimethylamino, Diethylamino.

**5.** Verfahren zur Herstellung von Aminosäureamid-Derivaten der allgemeinen Formel (I)

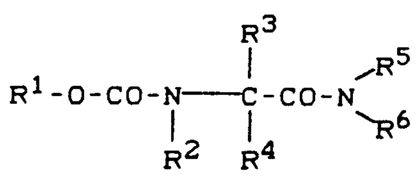

(I),

in welcher

$R^1$, $R^2$, $R^3$, $R^4$, $R^5$ und $R^6$ die in Anspruch 1 angegebene Bedeutung haben, dadurch gekennzeichnet, daß man

A) eine substituierte Aminosäure der Formel (II)

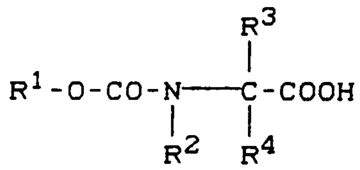

(II),

in welcher

$R^1$, $R^2$, $R^3$ und $R^4$ die oben angegebene Bedeutung haben, bzw. deren carboxyaktivierte Derivate,

gegebenenfalls in Gegenwart eines Katalysators, gegebenenfalls in Gegenwart eines Säurebindemittels und gegebenenfalls in Gegenwart eines Verdünnungsmittels

mit einem Amin der Formel (III)

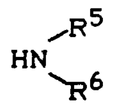

(III),

in welcher

$R^5$ und $R^6$ die oben angegebene Bedeutung haben, umsetzt, oder

B) substituierte Aminosäureamid-Derivate der Formel (IV)

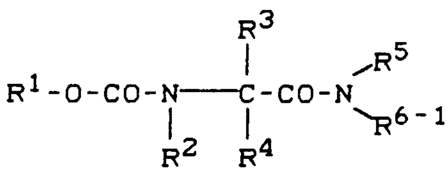

(IV),

in welcher

$R^1$, $R^2$, $R^3$, $R^4$ und $R^5$ die oben angegebene Bedeutung haben und

$R^{6-1}$ für unsubstituiertes oder substituiertes Phenyl steht, wobei als Phenylsubstituenten die für $R^6$ oben genannten Substituenten in Frage kommen,

in Gegenwart eines Katalysators, gegebenenfalls in Gegenwart eines Inertgases und in Gegenwart von Wasserstoff, gegebenenfalls in Gegenwart eines Verdünnungsmittels und gegebenenfalls unter Druck hydriert.

**6.** Schädlingsbekämpfungsmittel, gekennzeichnet durch einen Gehalt an mindestens einem Aminosäureamid-Derivat der Formel (I) nach den Ansprüchen 1 bis 5.

**7.** Verwendung von Aminosäureamid-Derivaten der Formel (I) nach den Ansprüchen 1 bis 5 zur Bekämpfung von Schädlingen.

**8.** Verfahren zur Bekämpfung von Schädlingen, dadurch gekennzeichnet, daß man Aminosäureamid-Derivate der Formel (I) nach den Ansprüchen 1 bis 5 auf Schädlingen und/oder ihren Lebensraum einwirken läßt.

**9.** Verfahren zur Herstellung von Schädlingsbekämpfungsmitteln, dadurch gekennzeichnet, daß man Aminosäureamid-Derivate der Formel (I) nach den Ansprüchen 1 bis 5 mit Streckmitteln und/oder oberflächenaktiven Mitteln vermischt.

## Claims

**1.** Amino acid amide derivatives of the general formula (I)

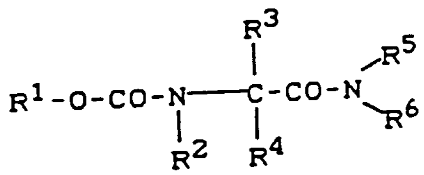

(I),

in which

$R^1$ represents alkyl, alkenyl, alkinyl, halogenoalkyl, halogenoalkenyl, halogeonalkinyl, cycloalkyl, cycloalkenyl, unsubstituted or substituted aryl, unsubstituted or substituted aralkyl, unsubstituted or substituted heteroaryl and unsubstituted or substituted heteroarylalkyl,

$R^2$, $R^5$ are identical or different and represent hydrogen or $C_1$-$C_4$-alkyl,

$R^3$, $R^4$ represent hydrogen, cycloalkyl or alkyl or

$R^3$ and $R^4$ together with the carbon atom to which they are bonded form a cycloalkyl ring,

$R^6$ represents unsubstituted or substituted straight-chain or branched cycloalkylalkyl, unsubstituted or substituted, straight-chain or branched cycloalkenylalkyl, or alkyl-bridged cycloalkylalkyl, with the exception of the compound N-(N-benzyloxycarbonyl-glycyl)-aminomethylcyclohexane.

**2.** Compounds of the formula (I), according to Claim 1, in which

$R^1$ represents straight-chain or branched alkyl having 1 to 6 carbon atoms, straight-chain or branched alkenyl or alkinyl having 2 to 6 carbon atoms, straight-chain or branched halogenoalkyl having 1 to 6 carbon atoms and 1 to 9 identical or different halogen atoms, straight-chain or branched halogenoalkenyl or halogenoalkinyl having 2 to 6 carbon atoms and 1 to 9 identical or different halogen atoms or substituted or unsubstituted cycloalkyl or cycloalkenyl having 3 to 7 carbon atoms, or represents in each case unsubstituted or substituted phenyl and pyridyl, or phenylalkyl which has 1 to 4 carbon atoms in the straight-chain or branched alkyl moiety and which is unsubstituted or substituted in the phenyl moiety, suitable substituents in the pyridyl or phenyl moiety in each case being:

alkyl, alkoxy and alkylthio, in each case having 1 to 4 carbon atoms; halogenoalkyl, halogenoalkoxy and halogenoalkylthio, in each case having 1 to 4 carbon atoms and 1 to 9 halogen atoms, the halogen atoms being identical or different; halogen; alkylalkoxy having 1 to 4 carbon atoms in each alkyl moiety; carbonylalkoxy having 1 to 4 carbon atoms in the alkyl moiety;

$R^2$ and $R^5$ represent hydrogen or methyl

$R^3$ and $R^4$ are identical or different and represent hydrogen, unsubstituted or substituted cycloalkyl having 3 to 7 carbon atoms or straight-chain or branched alkyl having 1 to 6 carbon atoms, or

$R^3$ and $R^4$ together with the carbon atom to which they are bonded form a cycloalkyl ring having 3 to 7 carbon atoms, and

$R^6$ represents unsubstituted or substituted cycloalkylalkyl having 3 to 7 carbon atoms in the cycloalkyl moiety and 1 to 6 carbon atoms in the straight-chain or branched alkyl moiety, or represents unsubstituted or substituted cycloalkenylalkyl having 5 to 8 carbon atoms in the cycloalkenyl moiety and 1 to 6 carbon atoms in the straight-chain or branched alkyl moiety, or represents cycloalkylalkyl which has 5 to 8 carbon atoms in the cycloalkyl moiety, which is unsubstituted or substituted and which is bridged by alkyl, having 1 to 4 carbon atoms in the bridging alkyl moiety and 1 to 6 carbon atoms in the straight-chain or branched alkyl moiety, suitable substituents being:

alkyl, alkoxy and alkylthio, in each case having 1 to 4 carbon atoms; halogenoalkyl,

halogenoalkoxy and halogenoalkylthio, in each case having 1 to 4 carbon atoms and 1 to 9 halogen atoms, the halogen atoms being identical or different; hydroxyl; dialkylamino having 1 to 4 carbon atoms per alkyl group; alkylalkoxy having 1 to 4 carbon atoms in each alkyl moiety.

**3.** Compounds of the formula (I), according to Claim 1, in which

$R^1$ represents straight-chain or branched alkyl having 1 to 4 carbon atoms, straight-chain or branched alkenyl or alkinyl having 2 to 4 carbon atoms, straight-chain or branched halogenoalkyl having 1 to 4 carbon atoms and 1 to 5 identical or different halogen atoms, straight-chain or branched halogenoalkenyl or halogenoalkinyl having 2 to 4 carbon atoms and 1 to 5 identical or different halogen atoms or unsubstituted or substituted cycloalkyl or cycloalkenyl having 3 to 6 carbon atoms, and furthermore represents phenyl which is unsubstituted or monosubstituted to trisubstituted by identical or different substituents, suitable substituents being the following:
alkyl, alkoxy and alkylthio, in each case having 1 to 4 carbon atoms; halogenoalkyl, halogenoalkoxy and halogenoalkylthio having 1 or 2 carbon atoms and 1 to 5 halogen atoms, the halogen atoms being identical or different; fluorine, chlorine, bromine and iodine; alkylalkoxy having 1 to 2 carbon atoms in each alkyl moiety; carbonylalkoxy having 1 or 2 carbon atoms in the alkyl moiety;

$R^2$ and $R^5$ represent hydrogen,

$R^3$ and $R^4$ are identical or different and represent hydrogen unsubstituted or substituted cycloalkyl having 3 to 6 carbon atoms or straight-chain or branched alkyl having 1 to 5 carbon atoms, or

$R^3$ and $R^4$ together with the carbon atom to which they are bonded form a cycloalkyl ring having 3 to 6 carbon atoms and

$R^6$ represents unsubstituted or substituted cycloalkylalkyl having 3 to 6 carbon atoms in the cycloalkyl moiety and 1 to 4 carbon atoms in the straight-chain or branched alkyl moiety, or represents unsubstituted or substituted cycloalkenylalkyl having 5 to 7 carbon atoms in the cycloalkenyl moiety and 1 to 4 carbon atoms in the straight-chain or branched alkyl moiety, or represents cycloalkylalkyl which has 5 to 7 carbon atoms in the cycloalkyl moiety, which is unsubstituted or substituted and which is bridged by alkyl, having 1 to 3 carbon atoms in the bridging alkyl moiety and 1 to 4 carbon atoms in the straight-chain or branched alkyl moiety, suitable substituents being:
alkyl, alkoxy and alkylthio, in each case having 1 to 4 carbon atoms; halogenoalkyl, halogenoalkoxy and halogenoalkylthio having 1 to 2 carbon atoms and 1 to 5 halogen atoms, the halogen atoms being identical or different; hydroxyl; dialkylamino having 1 to 2 carbon atoms per alkyl group; alkylalkoxy having 1 to 2 carbon atoms in each alkyl moiety.

**4.** Compounds of the formula (I), according to Claim 1, in which

$R^1$ represents methyl, ethyl, n- and i-propyl, n-, i-, s- and t-butyl, fluoromethyl, fluoroethyl, fluoropropyl, chloropropyl, fluorobutyl, chlorobutyl, difluoromethyl, difluoropropyl, dichloropropyl, difluorobutyl, dichlorobutyl, trifluoromethyl, trichloromethyl, trifluoroethyl, trichloroethyl, trifluoropropyl, trichloropropyl, trifluorobutyl, trichlorobutyl, allyl, butenyl, propargyl, butinyl, fluoro or chloroallyl, -butenyl, -propargyl, -butinyl, cyclopropyl, cyclopentyl, cyclohexyl, cyclopentenyl or cyclohexenyl, or represents phenyl which is unsubstituted or monosubstituted to disubstituted by identical or different substituents, suitable substituents being the following: methyl, ethyl, n- and i-propyl, n-, i-, s-and t-butyl, methoxy, ethoxy, i- and n-propoxy, n-, i-, s- and t-butoxy, trifluoromethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio; chlorine, bromine, fluorine, and

$R^2$ and $R^5$ represent hydrogen and

$R^3$ and $R^4$ are identical or different and represent hydrogen, methyl, ethyl, n- or i-propyl, n-, i-, s- or t-butyl, 3-pentyl, cyclopropyl, cyclopentyl or cyclohexyl or

$R^3$ and $R^4$ together with the carbon atom to which they are bonded form a cyclopropyl, cyclopentyl or cyclohexyl ring,

$R^6$ represents unsubstituted or substituted cycloalkylalkyl having 3, 5 or 6 carbon atoms in the cycloalkyl moiety and 1 to 3 carbon atoms in the straight-chain or branched alkyl moiety, or represents unsubstituted or substituted cycloalkenylalkyl having 5 to 7 carbon atoms in the cycloalkenyl moiety and 1 to 3 carbon atoms in the straight-chain or branched alkyl moiety, or represents unsubstituted or substituted cycloalkylalkyl which is bridged by alkyl and which has 5 to 7 carbon atoms in the cycloalkyl moiety, having 1 to 2 carbon atoms in the bridging alkyl moiety and 1 to 3 carbon atoms in the straight-chain or branched alkyl moiety, suitable substituents being the following:
methyl, ethyl, n- and i-propyl, n-, i-, s- and t-butyl, methoxy, ethoxy, i- and n-propoxy, n-, i-, s- and t-butoxy, trilfuormethyl, difluoromethyl, pentafluoroethyl, tetrafluoroethyl, trifluorochloroethyl, trifluoroethyl, trifluoroethoxy, difluoromethoxy, pentafluoroethoxy, tetrafluoroethoxy, trifluorochloroethoxy, trifluoromethoxy and trifluoromethylthio, hydroxyl, methylthio, ethylthio, n- and i-propylthio, dimethylamino, diethylamino.

**5.** Process for the preparation of amino acid amide derivatives of the general formula (I)

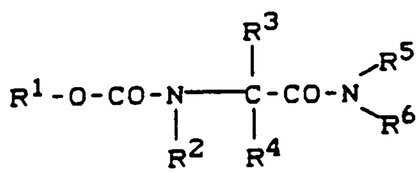

(I),

in which
$R^1$, $R^2$, $R^3$, $R^4$, $R^5$ and $R^6$ have the meaning given in Claim 1, characterised in that
A) a substituted amino acid of the formula (II)

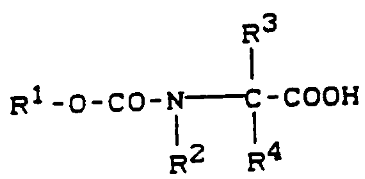

(II),

in which
$R^1$, $R^2$, $R^3$ and $R^4$ have the abovementioned meaning, or their carboxyl-activated derivatives, is reacted with an amine of the formula (III)

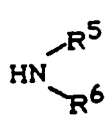

(III),

in which
$R^5$ and $R^6$ have the abovementioned meaning,
if appropriate in the presence of a catalyst, if appropriate in the presence of an acid-binding agent and if appropriate in the presence of a diluent, or

B) substituted amino acid amide derivatives of the formula (IV)

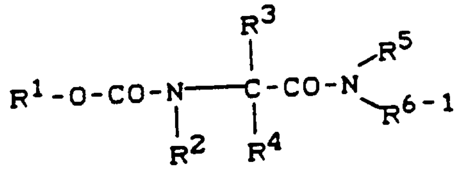
(IV),

in which
$R^1$, $R^2$, $R^3$, $R^4$ and $R^5$ have the abovementioned meaning and
$R^{6-1}$ represents unsubstituted or substituted phenyl, suitable phenyl substituents being the substituents mentioned above for $R^6$,
are hydrogenated in the presence of a catalyst, if appropriate in the presence of an inert gas and in the presence of hydrogen, if appropriate in the presence of a diluent and if appropriate under pressure.

**6.** Pesticides, characterised in that they contain at least one amino acid amide derivative of the formula (I) according to Claims 1 to 5.

**7.** Use of amino acid amide derivatives of the formula (I) according to Claims 1 to 5 for combating pests.

**8.** Method of combating pests, characterised in that amino acid amide derivatives of the formula (I) according to Claims 1 to 5 are allowed to act on pests and/or their environment.

**9.** Process for the preparation of pesticides, characterised in that amino acid amide derivatives of the formula (I) according to Claims 1 to 5 are mixed with extenders and/or surface-active agents.

**Revendications**

**1.** Dérivés d'amides d'amino-acides de formule générale (I)

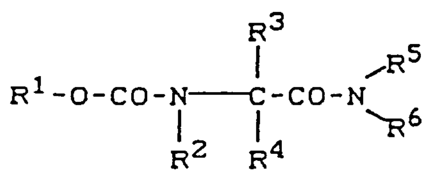
(I),

dans laquelle

$R^1$ est un groupe alkyle, alcényle, alcynyle, halogénalkyle, halogénalcényle, halogénalcynyle, cycloalkyle, cycloalcényle, aryle non substitué ou substitué, aralkyle non substitué ou substitué, hétéroaryle non substitué ou substitué et hétéroarylalkyle non substitué ou substitué,

$R^2$, $R^5$ sont identiques ou différents et représentent de l'hydrogène ou un groupe alkyle en $C_1$ à $C_4$,

$R^3$, $R^4$ représentent de l'hydrogène, un groupe cycloalkyle ou alkyle ou bien

$R^3$ et $R^4$ forment un noyau cycloalkyle conjointement avec l'atome de carbone auquel ils sont liés,

$R^6$ est un groupe cycloalkylalkyle non substitué ou substitué linéaire ou ramifié, un groupe cycloalcénylalkyle non substitué ou substitué linéaire ou ramifié, un groupe cycloalkylalkyle ponté par un radical alkyle,

excepté le N-(N-benzyloxycarbonylglycyl)-aminométhylcyclohexane.

**2.** Composés de formule (I) suivant la revendication 1, dans laquelle

$R^1$ est un groupe alkyle linéaire ou ramifié ayant 1 à 6 atomes de carbone, un groupe alcényle ou alcynyle linéaire ou ramifié ayant 2 à 6 atomes de carbone, un groupe halogénalkyle linéaire ou ramifié ayant 1 à 6 atomes de carbone et 1 à 9 atomes d'halogènes identiques ou différents, un groupe halogénalcényle ou halogénalcynyle linéire ou ramifié ayant 2 à 6 atomes de carbone et 1 à 9 atomes d'halogènes identiques ou différents ou un groupe cycloalkyle ou cycloalcényle substitué ou non substitué ayant 3 à 7 atomes de carbone, un groupe phényle et un groupe pyridyle dont chacun est non substitué ou substitué ou un groupe phénylalkyle non substitué ou substitué dans la partie phényle, ayant 1 à 4 atomes de carbone dans la partie alkyle linéaire ou ramifiée, en considérant comme substituants de la partie pyridyle ou de la partie phényle : des groupes alkyle, alkoxy et alkylthio ayant chacun 1 à 4 atomes de carbone ; des groupes halogénalkyle, halogénalkoxy et halogénalkylthio ayant chacun 1 à 4 atomes de carbone et 1 à 9 atomes d'halogènes, les atomes d'halogènes étant identiques ou différents ; un halogène ; un groupe alkylalkoxy ayant 1 à 4 atomes de carbone dans chaque partie alkyle ; un groupe carbonylalkoxy ayant 1 à 4 atomes de carbone dans la partie alkyle ;

$R^2$ et $R^5$ représentent de l'hydrogène ou un groupe méthyle,

$R^3$ et $R^4$ sont identiques ou différents et représentent de l'hydrogène, un groupe cycloalkyle non substitué ou substitué ayant 3 à 7 atomes de carbone ou un groupe alkyle linéaire ou ramifié ayant 1 à 6 atomes de carbone, ou bien

$R^3$ et $R^4$ forment, conjointement avec l'atome de carbone auquel ils sont liés, un noyau cycloalkyle de 3 à 7 atomes de carbone et

$R^6$ est un groupe cycloalkylalkyle non substitué ou substitué ayant 3 à 7 atomes de carbone dans la partie cycloalkyle et 1 à 6 atomes de carbone dans la partie alkyle linéaire ou ramifiée, un groupe cycloalcénylalkyle non substitué ou substitué ayant 5 à 8 atomes de carbone dans la partie cycloalcényle et 1 à 6 atomes de carbone dans la partie alkyle linéaire ou ramifiée, un groupe cycloalkylalkyle non substitué ou substitué ayant 5 à 8 atomes de carbone dans la partie cycloalkyle, ponté par un radical alkyle, avec 1 à 4 atomes de carbone dans la partie alkyle de pontage et 1 à 6 atomes de carbone dans la partie alkyle linéaire ou ramifiée, en considérant comme substituants : des groupes alkyle, alkoxy et alkylthio ayant chacun 1 à 4 atomes de carbone ; des groupes halogénalkyle, halogénalkoxy et halogénalkylthio ayant chacun 1 à 4 atomes de carbone et 1 à 9 atomes d'halogènes, les atomes d'halogènes étant identiques ou différents ; un groupe hydroxy ; un groupe dialkylamino ayant 1 à 4 atomes de carbone par groupe alkyle ; un groupe alkyl-alkoxy ayant 1 à 4 atomes de carbone dans chaque partie alkyle.

**3.** Composés de formule (I) suivant la revendication 1, dans lesquels

$R^1$ est un groupe alkyle linéaire ou ramifié ayant 1 à 4 atomes de carbone, un groupe alcényle ou alcynyle linéaire ou ramifié ayant 2 à 4 atomes de carbone, un groupe halogénalkyle linéaire ou ramifié ayant 1 à 4 atomes de carbone et 1 à 5 atomes d'halogènes identiques ou différents, un groupe halogénalcényle ou halogénalcynyle linéaire ou ramifié ayant 2 à 4 atomes de carbone et 1 à 5 atomes d'halogènes identiques ou différents ou un groupe cycloalkyle ou cycloalcényle non substitué ou substitué ayant 3 à 6 atomes de carbone et en outre un groupe phényle non substitué ou portant 1 à 3 substituants identiques ou différents, en considérant les substituants suivants :

des groupes alkyle, alkoxy et alkylthio ayant chacun 1 à 4 atomes de carbone : un groupe halogénalkyle ; des groupes halogénalkoxy et halogénalkylthio ayant 1 ou 2 atomes de carbone et 1 à 5 atomes d'halogènes, les atomes d'halogènes étant identiques ou différents ; du fluor, du chlore, du brome et de l'iode ; un groupe alkylalkoxy ayant 1 ou 2 atomes de carbone dans chaque partie alkyle ; un groupe carbonylalkoxy ayant 1 ou 2 atomes de carbone dans la partie alkyle ;

$R^2$ et $R^5$ représentent de l'hydrogène,

$R^3$ et $R^4$ sont identiques ou différents et représentent de l'hydrogène, un groupe cycloalkyle non substitué ou substitué ayant 3 à 6 atomes de carbone ou un groupe alkyle linéaire ou ramifié ayant 1 à 5 atomes de carbone, ou bien

$R^3$ et $R^4$ forment conjointement avec l'atome de carbone auquel ils sont liés un noyau cycloalkyle ayant 3 à 6 atomes de carbone et

$R^6$ est un groupe cycloalkylalkyle non substitué ou substitué ayant 3 à 6 atomes de carbone dans la partie cycloalkyle et 1 à 4 atomes de carbone dans la partie alkyle linéaire ou ramifiée, un groupe cycloalcénylalkyle non substitué ou substitué ayant 5 à 7 atomes de carbone dans la partie cycloalcényle et 1 à 4 atomes de carbone dans la partie alkyle linéaire ou ramifiée, un groupe cycloalkylalkyle non substitué ou substitué ayant 5 à 7 atomes de carbone dans la partie cycloalkyle et ponté par un radical alkyle, avec 1 à 3 atomes de carbone dans la partie alkyle de pontage et 1 à 4 atomes de carbone dans la partie alkyle linéaire ou ramifiée, en considérant comme substituants : des groupes alkyle, alkoxy et alkylthio ayant chacun 1 à 4 atomes de carbone ; un groupe halogénalkyle, des groupes halogénalkoxy et halogénalkylthio ayant 1 ou 2 atomes de carbone et 1 à 5 atomes d'halogènes, les atomes d'halogènes étant identiques ou différents ; un groupe hydroxy ; un groupe dialkylamino ayant 1 ou 2 atomes de carbone par groupe alkyle ; un groupe alkylalkoxy ayant 1 ou 2 atomes de carbone dans chaque partie alkyle.

**4.** Composés de formule (I) suivant la revendication 1, dans lesquels

$R^1$ est un groupe méthyle, éthyle, n-propyle, isopropyle, n-butyle, isobutyle, sec. -butyle, tertiobutyle, fluorométhyle, fluoréthyle, fluoropropyle, chloropropyle, fluorobutyle, chlorobutyle, difluorométhyle, difluoropropyle, dichloropropyle, difluorobutyle, dichlorobutyle, trifluorométhyle, trichlorométhyle, trifluoréthyle, trichloréthyle, trifluoropropyle, trichloropropyle, trifluorobutyle, trichlorobutyle, allyle, butényle, propargyle, butynyle, fluoro-ou chloro-allyle, -butényle, -propargyle, -butynyle, cyclopropyle, cyclopentyle, cyclohexyle, cyclopentényle ou cyclohexényle, un groupe phényle non substitué ou portant un ou deux substituants identiques ou différents, en considérant comme substituants : les groupes méthyle, éthyle, n-propyle, isopropyle, n-butyle, isobutyle, sec. -butyle, tertiobutyle, méthoxy, éthoxy, isopropoxy, n-propoxy, n-butoxy, isobutoxy, sec. -butoxy, tertio-butoxy, trifluorométhyle, difluorométhyle, pentafluoréthyle, tétrafluoréthyle, trifluorochloréthyle, trifluoréthyle, trifluoréthoxy, difluorométhoxy, pentafluoréthoxy, tétrafluoréthoxy, trifluorochloréthoxy, trifluorométhoxy et trifluorométhylthio ; le chlore, le brome, le fluor, et

$R^2$ et $R^5$ représentent de l'hydrogène et

$R^3$ et $R^4$ sont identiques ou différents et représentent l'hydrogène, un groupe méthyle, éthyle, n-propyle, isopropyle, n-butyle, isobutyle, sec. - butyle, tertiobutyle, 3-pentyle, cyclopropyle, cyclopentyle ou cyclohexyle, ou bien

$R^3$ et $R^4$ forment conjointement avec l'atome de carbone auquel ils sont liés un noyau cyclopropyle, cyclopentyle ou cyclohexyle,

$R^6$ est un groupe cycloalkylalkyle non substitué ou substitué ayant 3, 5 ou 6 atomes de carbone dans la partie cycloalkyle et 1 à 3 atomes de carbone dans la partie alkyle linéaire ou ramifiée, un groupe cycloalcénylalkyle non substitué ou substitué ayant 5 à 7 atomes de carbone dans la partie cycloalcényle et 1 à 3 atomes de carbone dans la partie alkyle linéaire ou ramifiée, un groupe cycloalkylalkyle non substitué ou substitué ayant 5 à 7 atomes de carbone dans la partie cycloalkyle et ponté par un radical alkyle, avec 1 ou 2 atomes de carbone dans la partie alkyle de pontage et 1 à 3 atomes de carbone dans la partie alkyle linéaire ou ramifiée, en considérant comme substituants : les groupes méthyle, éthyle, n-propyle, isopropyle, n-butyle, isobutyle, sec. -butyle, tertiobutyle, méthoxy, éthoxy, isopropoxy, n-propoxy, n-butoxy, isobutoxy, sec. -butoxy, tertiobutoxy, trifluorométhyle, difluorométhyle, pentafluoréthyle, tétrafluoréthyle, trifluorochloréthyle, trifluoréthyle, trifluoréthoxy, difluorométhoxy, pentafluoréthoxy, tétrafluoréthoxy, trifluorochloréthoxy, trifluorométhoxy, trifluorométhylthio, hydroxy, méthylthio, éthylthio, n-propylthio, isopropylthio, diméthylamino, diéthylamino.

**5.** Procédé de production de dérivés d'amides d'amino-acides de foule générale (I)

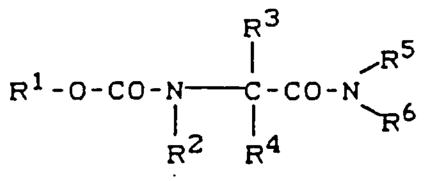

(I),

dans laquelle

$R^1$, $R^2$, $R^3$, $R^4$, $R^5$ et $R^6$ ont la définition indiquée dans la revendication 1,

caractérisé en ce que :

A) on fait réagir un amino-acide substitué de formule (II)

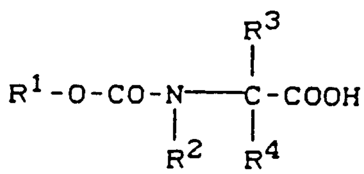

(II),

dans laquelle

$R^1$, $R^2$, $R^3$ et $R^4$ ont la définition indiquée ci-dessus ou leurs dérivés à groupes carboxy activés,

le cas échéant en présence d'un catalyseur, en la présence éventuelle d'un accepteur d'acide et le cas échéant en présence d'un diluant avec une amine de formule (III)

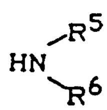

(III),

dans laquelle

$R^5$ et $R^6$ ont la définition indiquée ci-dessus, ou bien

B) on hydrogène des dérivés d'amides d'amino-acides substitués de formule (IV)

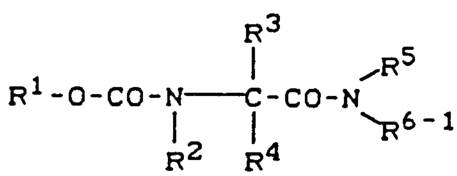

(IV),

dans laquelle

$R^1$, $R^2$, $R^3$, $R^4$ et $R^5$ ont la définition indiquée ci-dessus et

$R^{6-1}$ représente un groupe phényle non substitué ou substitué, en considérant comme substituants du groupe phényle les substituants mentionnés ci-dessus pour $R^6$,

en présence d'un catalyseur, éventuellement en présence d'un gaz inerte et en présence d'hydrogène, en la présence éventuelle d'un diluant et le cas échéant sous pression.

**6.** Compositions pesticides, caractérisées par une teneur en au moins un dérivé d'amide d'amino-acide de formule (I) suivant les revendications 1 à 5.

**7.** Utilisation de dérivés d'amides d'amino-acides de formule (I) suivant les revendications 1 à 5 pour combattre des parasites.

**8.** Procédé pour combattre des parasites, caractérisé en ce qu'on fait agir des dérivés d'amides d'amino-acides de formule (I) suivant les revendications 1 à 5 sur des parasites et/ou sur leur milieu.

**9.** Procédé de préparation de compositions pesticides, caractérisé en ce qu'on mélange des dérivés d'amides d'amino-acides de formule (I) suivant les revendications 1 à 5 avec des diluants et/ou des agents tensioactifs.